# FASCICULE DE BREVET EUROPEEN

(11) **EP 0 594 836 B1**
(45) Date de publication et mention de la délivrance du brevet: **21.05.1997**
(21) Numéro de dépôt: 93911834.5
(22) Date de dépôt: 28.04.1993
(51) Int. Cl.: G01L 9/08, G01L 9/00, A61M 5/168, A61B 5/021

(54) **DISPOSITIF DE MESURE DE PRESSION NOTAMMENT POUR INSTALLATION DE PERFUSION**
Druck-Messvorrichtung insbesondere für Perfusionsanlagen
PRESSURE MEASUREMENT DEVICE, IN PARTICULAR FOR A PERFUSION APPARATUS

(30) Priorité: 29.04.1992 FR 9205312
(43) Date de publication de la demande: 04.05.1994
(73) Titulaire: CHRONOTEC S.A.R.L., F-06600 Antibes (FR)
(72) Inventeur: HAUSER, Jean-Luc, F-06600 Antibes (FR)
(74) Mandataire: Bonneau, Gérard
(86) Numéro de dépôt international: FR9300416
(87) Numéro de publication internationale: WO9322641

(56) Documents cités:
- DE-A- 2 461 424
- DE-A- 2 809 264
- GB-A- 2 012 052
- US-A- 3 239 696
- IEEE TRANSACTIONS ON ELECTRON DEVICES vol. ED26, no. 12, Décembre 1979, N.Y., USA pages 1906-1910, J.M. BORKY ET AL. 'Integrated signal conditioning for silicon pressure sensors.'
- SIEMENS FORSCHUNGS- UND ENTWICKLUNGSBERICHTE vol. 10, no. 2, 1981, BERLIN, BRD pages 72 - 77, F. BREIMESSER ET AL. 'PIEZORESISTIVE PRESSURE SENSOR WITH SILICON DIAPHRAGM.'
- PATENT ABSTRACTS OF JAPAN vol. 7, no. 10 (P-168)(1155) 14 Janvier 1983 & JP-A-57 168 133 (RIKOO TOKEI K.K.) 16 October 1982
- SOVIET INVENTIONS ILLUSTRATED Section EI, Week 8726, 8 Juillet 1987 Derwent Publications Ltd., London, GB; Class S02, AN 87-184498 & SU,A,1 270 593 (LENGD MECH INST) 15 November 1986

## Description

### Domaine technique

La présente invention concerne les capteurs de pression et vise notamment de tels capteurs dans leur application à un dispositif de surveillance de la pression dans une installation de perfusion.

### Etat de la technique

On utilise des pompes à perfusion pour injecter des substances médicamenteuses à un patient, généralement par voie intravasculaire, mais également par d'autres voies d'accès appropriées.

Il est souhaitable de pouvoir contrôler la pression d'écoulement du fluide en aval de la pompe à perfusion, afin de détecter une éventuelle anomalie dans le fonctionnement de l'installation. En effet divers incidents peuvent survenir : la pompe peut s'emballer ou au contraire s'arrêter, le cathéter à travers lequel la substance médicamenteuse est injectée peut s'obturer progressivement par dépôt de sang, la ligne de perfusion peut être plicaturée, une fuite peut se produire en un point quelconque de la ligne de perfusion,etc...

On utilise actuellement pour surveiller la pression d'écoulement dans une ligne de perfusion des interrupteurs fonctionnant par tout ou rien et qui sont actionnés sous l'effet d'une variation importante de la pression par rapport à la pression nominale de fonctionnement . De tels dispositifs sont très peu précis, la précision de la mesure pouvant être de l'ordre de 50% de la valeur de la pression mesurée. Ils sont souvent utilisés simplement pour commander l'arrêt du moteur d'entraînement de la pompe lorsque la pression dépasse un certain seuil.

On connaît également d'autres dispositifs plus élaborés, tels que décrits dans les brevets US-A-4.446.344 et US-A-4.431.425. Les dispositifs décrits dans ces brevets comportent des interrupteurs qui sont capables non seulement d'arrêter le moteur lorsque la pression devient trop élevée mais qui remettent ce moteur en marche lorsque la pression est redescendue à une valeur acceptable. Cependant l'inconvénient de la précision insuffisante subsiste.

Un dispositif spécialement élaboré pour le domaine médical est décrit dans le brevet DE-A-2.461.424. Ce dispositif comporte une chambre remplie de glycérine chargée de transmettre à un capteur une pression s'exerçant sur la paroi de la chambre du capteur. Un tel dispositif utilisant un liquide (la glycérine) comme fluide de transmission de la pression est difficilement utilisable pour mesurer la pression dans une ligne de perfusion. En effet, de façon à réagir instantanément aux variations de pression dans une ligne de perfusion, il est nécessaire que la réponse du fluide de transmission soit linéaire, ce qui n'est pas le cas d'un liquide.

Un autre dispositif destiné à mesurer la pression dans un fluide se trouvant dans un récipient ou un conduit est décrit dans le brevet GB-A-2.012.052. Ce dispositif comprend un détecteur placé dans un fluide peu compressible délimité par une membrane en contact avec le fluide. Mais, dans ce dispositif, le capteur est directement fixé sur la membrane ce qui ne permet pas d'obtenir une réponse linéaire du capteur dans la mesure où la transmission de pression normalement effectuée par le fluide n'est pas parfaitement réalisée.

Le but de cette invention est donc de proposer un dispositif de mesure de pression qui permette d'effectuer en continu et avec une précision suffisante la mesure de la pression d'un fluide contenu dans une ligne de perfusion.

Un autre but de l'invention est de réaliser un dispositif de mesure de pression dans une ligne de perfusion utilisant un capteur présentant une courbe de réponse linéaire aux variations de pression

L'invention a donc pour objet un dispositif de mesure de pression comprenant un capteur de pression piezoélectrique, un boîtier comportant au moins une chambre contenant un fluide peu compressible et dans laquelle se trouve le capteur, la chambre étant délimitée sur une face par une membrane déformable en contact avec la paroi de la ligne de perfusion. Le fluide qui est interposé entre la membrane et le capteur est non liquide, a un coefficient de Poisson supérieur ou égal à 0,49 et un module instantané d'élasticité inférieur à 10 MPa de manière à ce que la courbe de réponse du capteur soit linéaire.

Suivant une autre caractéristique, le fluide est un gel silicone.

### Description de l'invention

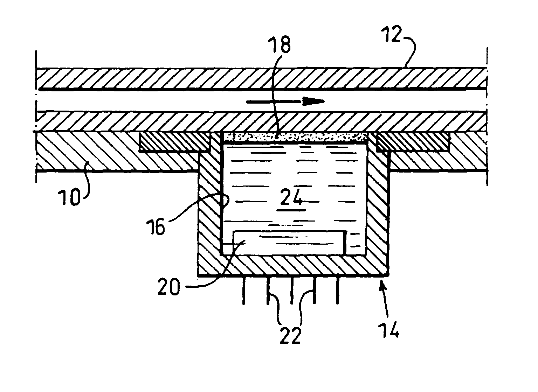

D'autres caractéristiques et avantages de l'invention ressortiront de la description qui va suivre, faite en se référant au dessin annexé, donné uniquement à titre d'exemple et, dont la figure unique une est vue en coupe, schématique, d'un capteur selon l'invention, associé à une ligne de perfusion.

On a représenté schématiquement au dessin une partie d'une installation de perfusion comprenant une pompe dont on a représenté une partie 10 de son boîtier et à partir de laquelle est alimentée une ligne de perfusion 12 qui peut être par exemple réalisée en silicone.

Un dispositif selon l'invention est utilisé pour mesurer en continu la pression d'écoulement dans la ligne de perfusion.

Ce dispositif comprend un boîtier 14 délimitant au moins une chambre 16 dont l'une des parois est constituée par une membrane déformable 18. Cette membrane peut être réalisée en un matériau élastomère ou en métal . Son élasticité peut provenir de la nature du matériau utilisé ou de la forme donnée à la membrane . C'est ainsi que cette dernière peut être constituée par un soufflet métallique . Elle peut être plane ou de forme cylindrique concave pour s'adapter sur la ligne de perfusion, ou encore légèrement bombée vers l'extérieur, ce qui peut permettre d'améliorer la sensibilité du dispositif de mesure. Elle est collée au boîtier ou fixée à ce dernier par tout moyen approprié.

Dans le fond de la chambre 16 est disposé un capteur piézoélectrique 20 connu en soi, qui peut être de tout type convenable, résistif ou capacitif, àmesure de pression absolue ou relative.

A titre d'exemple, un tel capteur peut comporter une plaque en silicium, associé à un montage électrique en pont de Wheatstone, comportant par exemple cinq conducteurs 22 de connexion .

Entre la membrane et le capteur piézoélectrique est disposé un fluide de remplissage 24. Le rôle de ce fluide de remplissage est tout à fait essentiel car il assure la transmission de la pression depuis la membrane 18 jusqu'au capteur piézoélectrique 20. Il faut pouvoir réagir instantanément à toute variation de pression dans la ligne de perfusion qui pourrait, entre autres, faire courir un risque au patient. Cette réaction instantanée exige que la réponse du capteur soit linéaire, sinon toute dérive dans la courbe de réponse sera difficile à interpréter avec une réponse non linéaire. Pour satisfaire à cette exigence de linéarité, le fluide de remplissage de la chambre doit être incompressible ou quasi incompressible et présenter un très bas module d'élasticité. Un tel matériau, qui ne peut pas être un liquide, doit ainsi avoir un coefficient de Poisson supérieur ou égal à 0.49, un module instantané d'élasticité inférieur à 10 MPa, et une viscosité de l'ordre de quelques centaines de centipoises.

Un exemple d'un tel matériau peut être constitué par un gel silicone tel qu'on en applique sur la peau d'un patient pour transmettre un signal échographique d'un transducteur à travers ce patient. On citera comme exemples le "Silicone gel system Q7 -2218" de la Société Dow Corning et le " Silicone gel System Q7 - 2167" de la même société, associé au "Silicone gel system Q7 - 2168".

Un tel dispositif est particulièrement adapté pour être utilisé avec une pompe à perfusion portable ambulatoire, étant donné sa dimension et son poids très faibles et le fait qu'il permet de mesurer en continu l'évolution de la pression en aval de la pompe de façon précise, fiable et linéaire.

La précision peut être de l'ordre de 1% de la valeur de la pression mesurée. La mesure est linéaire grâce à l'utilisation d'un capteur piézoélectrique et à l'utilisation d'un matériau approprié pour transmettre les variations de pression de la membrane à ce capteur.

Il permet de détecter l'absence de liquide dans la ligne de perfusion, en cas de fuite ou de réservoir vide .

Il permet de surveiller les variations de pression dans l'installation de perfusion. En cas d'obstruction progressive par un dépôt sanguin d'un cathéter vasculaire, il permet de déceler l'augmentation de pression correspondante. On est ainsi averti avant l'obstruction complète du cathéter, ce qui permet d'intervenir en temps utile pour déclencher une opération de nettoyage du conduit. On évite ainsi l'explantation et le remplacement du cathéter.

Un tel dispositif peut être associé à une unité de commande électronique gérant le fonctionnement global de la pompe. L'information fournie par le capteur est alors utilisée pour commander et ajuster le débit de la pompe, ce que ne permettent pas de réaliser les interrupteurs connus fonctionnant par tout ou rien.

On notera également parmi les autres avantages de ce dispositif le fait que le capteur n'est pas en contact direct avec le fluide contenu dans la ligne de perfusion.

## Revendications

1. Dispositif de mesure de pression dans une ligne de perfusion (12) comprenant un capteur de pression piezoélectrique (20), un boîtier (14) comportant au moins une chambre (16) contenant un fluide (24) peu compressible,et dans laquelle se trouve placé ledit capteur, ladite chambre étant délimitée sur une face par une membrane déformable (18) en contact avec la paroi de ladite ligne de perfusion; ledit dispositif étant caractérisé en ce que ledit fluide est interposé entre ladite membrane et ledit capteur, est non liquide et a un coefficient de Poisson supérieur ou égal à 0,49 et un module instantané d'élasticité inférieur à 10 MPa de manière à ce que la courbe de réponse dudit capteur soit linéaire.

2. Dispositif selon la revendication 1, caractérisé en ce que ledit fluide (24) a une viscosité de l'ordre de quelques centaines de centipoises.

3. Dispositif selon la revendication 1 ou 2, caractérisé ou ce que ledit fluide est un gel silicone.

4. Dispositif selon l'une quelconque des revendications précédentes, caractérisé en ce que ladite membrane déformable (18) est bombée vers l'extérieur.

## Patentansprüche

1. Vorrichtung zur Messung des Drucks in einer Infusionsleitung (12), umfassend einen piezoelektrischen Druckfühler (20), ein Gehäuse (14), das mindestens eine Kammer (16) aufweist, die ein wenig komprimierbares Fluid (24) enthält und in der dieser Fühler angeordnet ist, wobei diese Kammer auf einer Seite durch eine verformbare Membran (18) abgegrenzt ist, die mit der Wand dieser Infusionsleitung in Kontakt ist, wobei diese Vorrichtung dadurch gekennzeichnet ist, daß das Fluid zwischen der Membran und dem Fühler angeordnet ist, nicht flüssig ist und einen Poissonkoeffizienten größer als oder gleich 0,49 und einen momentanen Elastizitätsmodul kleiner als 10 MPa hat, so daß die Ansprechkurve des Fühlers linear ist.

2. Vorrichtung nach Anspruch 1, dadurch gekennzeichnet, daß das Fluid (24) eine Viskosität von etwa einigen Hundert Zentipoise hat.

3. Vorrichtung nach Anspruch 1 oder 2, dadurch gekennzeichnet, daß das Fluid ein Silicongel ist.

4. Vorrichtung nach einem der vorhergehenden Ansprüche, dadurch gekennzeichnet, daß die verformbare Membran (18) nach außen gewölbt ist.

## Claims

1. Pressure measurement device in an infusion tube (12)including a pressure sensor (20), a housing (14) comprising at least one chamber (16) containing a little compressible fluid (24) and wherein is located said sensor, said chamber being delimited on one side by a deformable membrane (18) contacting the wall of said infusion tube, said device being characterized in that said fluid is interposed between said membrane and said sensor, is non-liquid and has a Poisson ratio of at least 0.49 as well as an instantaneous modulus of elasticity of under 10 MPa so that the sensor response curve is linear.

2. Device according to claim 1, characterized in that said fluid (24) has a viscosity of the order of hundreds of centipoises.

3. Device according to any one of claims 1 to 3, characterized in that said fluid is a silicone gel.

4. Device according to any one of the preceding claims, characterized in that said deformable membrane (18) is convex in form.
